(19) Europäisches Patentamt
European Patent Office
Office européen des brevets

(11) Publication number: 0 105 944
A1

(12) # EUROPEAN PATENT APPLICATION

(21) Application number: 82109333.3

(22) Date of filing: 08.10.82

(51) Int. Cl.³: C 07 D 263/12

(43) Date of publication of application: 25.04.84
Bulletin 84/17

(84) Designated Contracting States: BE DE FR GB IT NL

(71) Applicant: THE DOW CHEMICAL COMPANY, Dow Center 2030 Abbott Road Post Office Box 1967, Midland Michigan 48640 (US)

(72) Inventor: Kaiser, Mark Edward, 2808 Blairmont, Midland Michigan, 48640 (US)
Inventor: Larson, David Leslie, Route 10 Box 217, Cookeville Tennessee, 38501 (US)

(74) Representative: Casalonga, Axel et al, BUREAU D.A. CASALONGA OFFICE JOSSE & PETIT
Baaderstrasse 12-14, D-8000 München 5 (DE)

(54) **Liquid phase preparation of 2-substituted-2-oxazolines with organic zinc salt catalysts.**

(57) 2-Substituted-2-oxazolines are prepared by contacting N-(2-hydroxyalkyl)carboxamides with a catalytic amount of an organic zinc salt at an elevated temperature.

EP 0 105 944 A1

# LIQUID PHASE PREPARATION OF 2-SUBSTITUTED-2-OXAZOLINES WITH ORGANIC ZINC SALT CATALYSTS

2-Substituted-2-oxazolines form a known class of compounds having many members and many utilities. The chemistry of such oxazolines has been summarized, for example, by Wiley et al., Chemical Reviews, Vol. 44, 447 (1949), by Seeliger et al., Angew. Chem. International Edition, Vol. 5, No. 10, 875 (1966), and by Frump, Chemical Reviews, Vol. 71, No. 5, 483 (1971).

One of the prior art processes for preparing 2-substituted-2-oxazolines is the cyclodehydration of N-(2-hydroxyalkyl)carboxamides. This cyclodehydration occurs in vapor phase over alumina (U.S. Patent 3,562,263 and Frump, as mentioned above, page 485) and in liquid phase over certain salts of manganese, cobalt, rare earth metals, molybdenum and tungsten (U.S. Patents 3,681,329 and 3,681,333). The latter two patents indicate that the cyclodehydration reaction is brought about by heating the hydroxyamide and catalyst together in a distillation apparatus from which the oxazoline product distills from the reaction mixture as it is formed along with the by-product water.

In another process, 2-substituted-2-oxazolines are prepared by heating N-(2-hydroxyalkyl)carboxamides in the presence of certain inorganic iron salts (European Patent Publication 0033752, published August 19, 1981, The Dow Chemical Company). However, this process does not result in as high yields as does the invention described in this application.

The present invention is a cyclodehydration process for making a 2-substituted-2-oxazoline characterized by reacting in the liquid phase an N-(2-hydroxyalkyl)-carboxamide having the formula

$$R-\overset{O}{\overset{\|}{C}}-NH-CH_2CH_2OH$$

where R is alkyl of 1 to 17 carbon atoms, **optionally substituted phenyl, aralkyl of 7 to 17 carbon atoms, alkenyl of 2 to 17 carbon atoms, cycloalkyl of 3 to 17 carbon atoms or a carboxylic acid/amine salt precursor of said carboxamide.**

The temperature/pressure relationship is normally adjusted such that the oxazoline and water codistill from the reaction mixture essentially as fast as they are formed.

The N-(2-hydroxyalkyl)carboxamides used in the instant process have the formula

$$I. \quad R-\overset{O}{\overset{\|}{C}}-NH-CH_2-CH_2-OH$$

wherein R is alkyl of 1 to 17 carbon atoms, or phenyl, or a carboxylic acid/amine salt precursor of said carboxamide. Such carboxamides are typically prepared

by reacting a carboxylic acid or a lower alkyl ester of the carboxylic acid with an ethanolamine of the formula

II. $NH_2-CH_2-CH_2-OH$ .

The carboxylic acid/amine salt which is formed initially in these reactions can be used in the instant process in place of the carboxamide. When such carboxylic acid/amine salts are used, the carboxamide is generated in situ. R in formula I is preferably methyl, ethyl or phenyl and more preferably methyl or ethyl. Examples of suitable N-(2-hydroxyalkyl)-carboxamides include compounds of formula I having the following values for R: $CH_3$, $C_2H_5$, $C_3H_7$, $C_7H_{15}$, $C_9H_{19}$, $C_{11}H_{23}$, $C_{17}H_{35}$, $C_6H_5$, $C_6H_4CH_3$, $C_6H_5CH_2$, $CH_3(CH_2)_7CH=CH(CH_2)_7$ and cyclohexyl.

The catalysts in the instant cyclodehydration reaction are organic zinc salts which are soluble in the carboxamide reactant or liquid reaction medium. The term "soluble" is not meant to imply that the zinc salt is soluble or miscible in all proportions with the carboxamide or liquid reaction medium, but instead has at least a minimum solubility (e.g., at least about 100 parts per million or more) at reaction temperature. Such zinc salts are used in the process in catalytic amounts. Normally, the zinc salts are charged in amounts of from 0.005 to 0.4 mole of zinc salt per mole of carboxamide reactant, but more or less of the zinc salts can be used, if desired. Carboxylic acid salts of zinc having the formula $(RCOO^-)_2Zn^{++}$ wherein R is a $C_1$-$C_{20}$ aliphatic or alicyclic radical or an inertly-substituted $C_1$-$C_{20}$ aliphatic or alicyclic carboxylic acid radical may be used as catalysts in the invention.

Suitable carboxylic acid zinc salts include, for example, zinc acetate, zinc formate, zinc propionate, zinc stearate and zinc neodecanoate. Zinc acetate is the current preferred catalyst.

The instant cyclodehydration reaction may be conducted neat or in solution with a suitable inert solvent. By "inert" is meant inert in the process. Suitable such inert solvents include, for example, chlorinated hydrocarbon solvents, aromatic hydrocarbons, cycloaliphatic hydrocarbons and aliphatic hydrocarbons. It is preferred however, to conduct the reaction neat.

The reaction temperature must, obviously, be sufficient to promote the cyclodehydration reaction and is normally selected in the range of from about 140°C to about 280°C. Preferred reaction rates have been observed at temperatures of from about 160°C to about 250°C. The instant cyclodehydration reaction is also preferably conducted under reduced pressure. This facilitates product recovery in that frequently a reaction temperature may be chosen which is above the boiling point of the 2-substituted-2-oxazoline product and below the boiling point of the N-(2-hydroxyalkyl)carboxamide. In this manner, the 2-substituted-2--oxazoline can be removed from the reaction mixture as a volatile gas essentially as fast as it is formed. This is very desirable since the instant cyclodehydration reaction is a reversible process and by removing the products, the reaction is forced to completion by substantially reducing the reverse reaction. Water normally codistills with the 2-substituted-2-oxazoline product.

The instant process may be conducted in a batch process or by a continuous process. In the preferred continuous process the N-(2-hydroxyalkyl)carboxamide reactant

is metered into the reaction vessel at essentially the same rate as the 2-substituted-2-oxazoline and water are removed.

The following examples further illustrate the invention.

Example 1 - Preparation of 2-Ethyl-2-Oxazoline Over Zinc Acetate Dihydrate

Zinc acetate dihydrate (10.0 g; 0.045 mole) and 95.4 percent pure N-(2-hydroxyethyl)propionamide (20.0 g; 0.162 mole) were charged to a reaction vessel equipped with a stirring means, a metering pump, and a 5-plate distillation column with a take-off head. The pressure over the reaction mixture was adjusted to 50 mm Hg and the reaction mixture heated to 200°C. The reaction mixture was held at 200°C and 95.4 percent pure N-(2-hydroxyethyl)propionamide (290 g; 2.35 mole) was pumped in at approximately 0.9 g/min to the system. As the propionamide was added to the reaction mixture, a water-white distillate was collected overhead through the distillation apparatus at a head temperature of 40°C-45°C. After the addition of the propionamide was complete, the pot was heated to 220°C to drive off the last amounts of 2-ethyl-2-oxazoline. The overhead distillate temperature reached a maximum of 41°C during this post--heating step. A total of 294.8 g of water-white distillate was thus obtained overhead leaving 21.7 g of a tan, wet paste remaining in the pot. Analysis of the distillate overheads by gas chromatography using an internal standard and a Karl Fischer water titration showed the material to be 2-ethyl-2-oxazoline, water and very minor amounts of

unreacted propionamide and 2-methyl-2-oxazoline. The impurities in the propionamide reactant were: water (approximately 1 percent); monoethanolamine (approximately 2-3 percent); and the amidoester of propionic acid and monoethanolamine (approximately 1 percent).

The oxazoline was produced in 96.2 percent yield, based on the pure N-(2-hydroxyethyl)propionamide charged to the system. The amount of water produced according to analysis was 93.0 percent of theory. The 2-ethyl-2-oxazoline can be easily separated from the mixture by selective extraction using diethylbenzene followed by distillation.

Example 2

In another experiment, 2-ethyl-2-oxazoline was prepared in 82 percent yield by warming a propionic acid/-ethanolamine salt in the presence of approximately 2 mole percent zinc acetate dihydrate at a temperature of 200°C/50 mm Hg. This was a batch experiment in which the acid/amine salt and catalyst were initially charged and warmed to the indicated reaction temperature. There was a pause in the rise in temperature during which the acid/amine salt was converted to the amide. Otherwise, this reaction proceeded the same as Example 1 above. The product was similarly recovered as an overhead distillate with water.

Other catalysts and carboxamide reactants as set forth above could be similarly used to produce the 2-substituted-2-oxazolines.

1. A cyclodehydration process for making a 2-substituted-2-oxazoline characterized by reacting in the liquid phase an N-(2-hydroxyalkyl)carboxamide having the formula

$$R-\overset{\overset{O}{\parallel}}{C}-NH-CH_2CH_2OH$$

where R is alkyl of 1 to 17 carbon atoms, **optionnaly substituted phenyl, aralkyl of 7 to 17 carbon atoms, alkenyl of 2 to 17 carbon atoms, cycloalkyl of 3 to 17 carbon atoms or a carboxylic acid/amine salt precursor of said carboxamide with a catalytic amount of an organic zinc salt.**

2. The process of Claim 1 wherein R is methyl, ethyl or phenyl.

3. The process of Claim 1 wherein R is methyl or ethyl.

4. The process of Claim 1 wherein said catalyst is charged in amounts of from 0.005 to 0.4 mole of organic zinc salt per mole of carboxamide reactant.

5. The process of Claim 1 wherein said catalyst is zinc acetate or zinc neodecanoate.

6. The process of Claim 1 wherein the process is conducted under conditions of temperature and pressure such that the 2-substituted-2-oxazoline product is removed from the reaction mixture as a volatile gas essentially as it is formed.

**7. The process of Claim 1 wherein the process is conducted at a temperature in the range from about 140 degree C to 280 degree C and preferably in the range from about 160 degree C to 250 degree C under reduced pressure.**

0105944

European Patent Office

# EUROPEAN SEARCH REPORT

Application number

EP 82 10 9333

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| X | JOURNAL OF THE CHEMICAL SOCIETY CHEMICAL COMMUNICATIONS, 1972, pages 639-640, London, GB. E. GHERA et al.: "Formation of delta2-oxazolines by cyclization of N-acyl-beta-hydroxy-amines on zinc acetate. An oxazoline complex of zinc acetate" * Whole article * --- | 1-4 | C 07 D 263/12 |
| X | US-A-3 741 961 (J. E. KMIECIK) * Claims * --- | 1 | |
| D,A | EP-A-0 033 752 THE DOW CHEMICAL CY.) * Claims * --- | 1 | |
| A | FR-A-2 160 126 (BAYER AG.) * Claims * ----- | 1 | TECHNICAL FIELDS SEARCHED (Int. Cl. 3) |
| | | | C 07 D 263/12 |

The present search report has been drawn up for all claims

| Place of search | Date of completion of the search | Examiner |
|---|---|---|
| THE HAGUE | 08-06-1983 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82